# EUROPÄISCHE PATENTSCHRIFT

(11) **EP 1 667 673 B1**
(45) Veröffentlichungstag und Bekanntmachung des Hinweises auf die Patenterteilung: **11.09.2013**
(21) Anmeldenummer: 04765640.0
(22) Anmeldetag: 27.09.2004
(51) Int. Cl.: A61K 31/202, A61P 3/10, A61K 36/54, A61K 36/55, A61K 36/535, A23L 1/30

(54) **Alpha-Linolensäurehaltige Pflanzenöle gegen Diabetes**
Alpha-linolenic acid-containing vegetable oils used against diabetes
Huiles vegetales contenant de l'acide alpha-linolénique contre le diabète

(30) Priorität: 30.09.2003 DE 10345401
(43) Veröffentlichungstag der Anmeldung: 14.06.2006
(73) Patentinhaber: Dr. Willmar Schwabe GmbH & Co. KG, 76227 Karlsruhe (DE)
(72) Erfinder: KÖHLER, Stephan, 68723 Schwetzingen (DE); TRAN, Cam-Tuan, 76676 Graben-Neudorf (DE); KOCH, Egon, 76229 Karlsruhe (DE)
(74) Vertreter: Adam, Holger
(86) Internationale Anmeldenummer: PCT/EP2004/010817
(87) Internationale Veröffentlichungsnummer: WO 2005/032539

(56) Entgegenhaltungen:
- EP-A- 0 145 873
- DE-A- 10 029 562
- DATABASE WPI Section Ch, Week 200438 Derwent Publications Ltd., London, GB; Class B04, AN 2004-401622 XP002312629 & CN 1 483 453 A (LU Y) 24. März 2004 (2004-03-24)
- COSTE T C ET AL: "Supplementations with polyunsaturated fatty acids in diabetic neuropathy" CAHIERS DE NUTRITION ET DE DIETETIQUE 2004 FRANCE, Bd. 39, Nr. 3, 2004, Seiten 185-194, XP008041104 ISSN: 0007-9960
- DATABASE WPI Section Ch, Week 199641 Derwent Publications Ltd., London, GB; Class B05, AN 1996-408310 XP002312630 & JP 08 198749 A (MORISHITA ROUSSEL KK) 6. August 1996 (1996-08-06)
- RACCAH D ET AL: "Polyunsatured fatty acids and diabetes" CAHIERS DE NUTRITION ET DE DIETETIQUE 1997 FRANCE, Bd. 32, Nr. 6, 1997, Seiten 349-358, XP008041101 ISSN: 0007-9960
- IKEDA A ET AL: "Effects of intravenous perilla oil emulsion on nutritional status, polyunsaturated fatty acid composition of tissue phospholipids, and thromboxane A2 production in streptozotocin-induced diabetic rats" NUTRITION 1995 UNITED STATES, Bd. 11, Nr. 5, 1995, Seiten 450-455, XP008041110 ISSN: 0899-9007
- MESHCHERYAKOVA V A ET AL: "Comparative evaluation of influence of a diet with includion of "eiconol" or linseed oil on some parameters of a lipid metabolism in the patients with diabetes 2 types" VOPROSY PITANIYA, Bd. 70, Nr. 2, 2001, Seiten 28-31, XP008041105 ISSN: 0042-8833
- MCMANUS R M ET AL: "Comparison of effects of omega-3 fatty acids of linseed and fish oil origin in type II diabetes" CLINICAL AND INVESTIGATIVE MEDICINE, Bd. 16, Nr. 4 SUPPL., 1993, Seite B34, XP008041107 & ANNUAL MEETING OF THE CANADIAN SOCIETY FOR CLINICAL INVESTIGATION AND THE ROYAL COLLEGE OF PHYSICIAN; VANCOUVER, BRITISH COLUMBIA, CANADA; SEPTEMBER 9-13, 1993 ISSN: 0147-958X
- BECKERMANN: "Vergleich der Bioverfügbarkeit von Eicosapentaensäure und Docosahexaensäure...", ARZNEIMITTEL-FORSCHUNG/DRUG RES., vol. 40, no. 6, 1990, pages 700-704,

## Beschreibung

Die Erfindung betrifft die Verwendung von α-linolensäurehaltigen Pflanzenölen in gebundener Form in Form von Triglyceriden, optional in Kombination mit Zimt oder einem Extrakt aus Zimtrinde, zur Prophylaxe, Behandlung oder zur Unterstützung der Behandlung von Krankheiten, die mit einem erhöhten Gehalt an Glucose und / oder HbA_{1c} im Blut einhergehen, nämlich von Diabetes mellitus Typ 2, insbesondere diabetischer Retinopathie, Makulopathie und Neuropathie und des metabolischen Syndroms.

Bei Diabetes mellitus handelt es sich um eine Gruppe von Erkrankungen, die durch hohe Blutglucose-Spiegel gekennzeichnet sind und die durch eine unzureichende Insulinproduktion und/oder Insulinaktivität verursacht werden. Die erhöhten Blutglucose-Spiegel werden von metabolischen Funktionsstörungen und speziell einer verstärkten Glykosylierung von Proteinen begleitet. Der Nachweis von glykosyliertem Hämoglobin (HbA_{1c}) wird deshalb auch zur langfristigen Kontrolle des Blutglucosespiegels bei Diabetes-Patienten verwendet. Als Folge der Stoffwechselstörungen kommt es häufig zu einer beschleunigten Ausbildung von atherosklerotischen Gefäßveränderungen, die mit Durchblutungsstörungen wie Herzinfarkt, Herzversagen, Schlaganfall oder Nekrosen der unteren Extremitäten einhergehen. Darüber hinaus ist Diabetes mellitus eine wesentliche Ursache von Erblindungen, Neuropathien und Nierenversagen. Studien haben ergeben, dass die Todesrate von Menschen mittleren Alters mit Diabetes mellitus doppelt so hoch ist wie die gesunder Menschen.

Bei Diabetes mellitus unterscheidet man im Wesentlichen zwischen zwei verschiedenen Formen: Typ 1 (insulinabhängiger oder jugendlicher Diabetes) und Typ 2 (insulinunabhängiger oder Altersdiabetes). Diabetes mellitus Typ 1 wird vermutlich durch eine autoimmune Schädigung der Insulin-produzierenden Beta-Zellen im Pankreas ausgelöst. Die Behandlung beruht deshalb vor allem auf der Verabreichung von lmmunsuppressiva und der Substitution von Insulin. Im Gegensatz dazu ist Diabetes mellitus Typ 2 durch Insulinresistenz gekennzeichnet, die durch unzureichendes Ansprechen der Skelettmuskulatur, der Leber und des Fettgewebes auf Insulin erklärt werden kann. Insulinrestistenz wird heute als eine von mehreren Erscheinungsformen des metabolischen Syndroms (Syndrom X) angesehen, das neben einer beeinträchtigten Glucosetoteranz häufig mit Bluthochdruck, Fettstoffwechselstörungen, sowie koronaren, cerebralen und peripheren Gefäßerkrankungen einhergeht. Es herrscht heute weitgehende Übereinstimmung, dass das metabolische Syndrom durch eine Kombination von genetischen und hormonellen Faktoren sowie durch die Lebensführung (Übergewicht, Bewegungsmangel und Fehlernährung) verursacht wird.

Diabetes mellitus gehört weltweit zu den häufigsten Krankheiten. Schätzungen gehen davon aus, dass etwa 120 Millionen Menschen an Diabetes leiden, wobei bis zum Jahre 2010 mit einer Verdopplung dieser Zahl gerechnet wird. Mehr als 90 % davon sind dem Diabetes mellitus Typ 2 zuzuordnen. Die bisher zur Verfügung stehenden Behandlungsmethoden zielen vor allem auf eine Senkung des erhöhten Blutglucosespiegels und einer Verbesserung der Insulinresistenz. Wie klinische Studien gezeigt haben, ist eine strikte Kontrolle des Blutglucosespiegels besonders wichtig, weil dadurch die Entstehung von Folgekrankheiten wirksam verhindert werden kann. Neben Umstellungen in der Lebensführung ist vor allem die medikamentöse Therapie mit Sulfonylharnstoffen, alpha-Glucosidasehemmern, Thiazolidinedionen und Meglitiniden üblich. Die meisten dieser Medikamente verursachen aber mehr oder weniger schwerwiegende Nebenwirkungen. Außerdem entwickelt sich häufig eine Toleranz gegenüber diesen Arzneimitteln oder es kommt zu lebensbedrohlichen hypoglykämischen Episoden.

Gemeinsamer auslösender Faktor der bei Erhöhung des Blutzuckerspiegels (Hyperglykämie) häufig auftretenden Retino- und Makulopathie sowie von Neuropathien sind die durch die Hyperglykämie hervorgerufenen langfristigen biochemischen und zellphysiologischen Veränderungen, die schließlich zu einer Netzhaut- und Nervenschädigung führen. An diesem Prozeß sind maßgeblich AGEs (advanced glycosylation end products) beteiligt: AGEs sind ein Folgeprodukt der nichtenzymatischen Glykosilierung von Proteinen bei erhöhten Glukosespiegeln. Die Glykosilierung fördert die irreversible Vernetzung von ins Gewebe ausgetretenen Plasmaproteinen mit Gewebeproteinen der Kapillaren (z. B. in der Retina). Hierdurch kommt es zu Verdickungen und funktionellen Veränderungen der Basalmembran. Die lange Lebensdauer der AGEs erklärt unter anderem, dass Gefäßveränderungen noch lange nach Normalisierung der Blutzuckerspiegel fortschreiten können. Außerdem wird bei erhöhten Blutzuckerspiegeln vermehrt das enzym Aldosereduktase produziert, um Glukose abzubauen. Beim Aldosereduktasevermittelten Glukoseabbau entsteht in der Zelle vermehrt Sorbit (ein Polyol), das anschließend durch die Sorbitdehydrogenase in Fruktose überführt wird. Als Folge der Hyperglykämie kommt es zu einer vermehrten intrazellulären Anreicherung von Sorbit und Fruktose, die beide in hohen Konzentrationen toxisch wirken. Sowohl die Hyperglykämie als auch der vermehrt beanspruchte Polyolweg hemmen ferner die natriumabhängige Myoinositolaufnahme. Durch das Übergewicht anderer osmotisch wirksamer Substanzen kommt es überdies zu einer kompensatorischen Hemmung der zellulären Myoinositolsynthese, was zu einer Schädigung der Myelinscheiden von Nervenzellen führen kann.

Eine Schlüsselrolle bei der Entstehung der diabetischen Neuropathie scheint nach neuen Erkenntnissen die Proteinkinase C (PKC) zu spielen. Hyperglykämie und andere Mechanismen führen bei Diabetes mellitus zu einer Aktivierung von PKC, die ihrerseits strukturelle Veränderungen in den endoneuralen Gefäßen induziert. Es wird angenommen, dass PKC über den in der Folge reduzierten nervalen Blutfluss zur diabetischen Neuropathie beiträgt.

Erhöhte Blutzuckerspiegel verändern überdies die Autoregulation der retinalen Zirkulation. Durch den vermehrten Blutfluss und die bei Diabetes erhöhte Viskosität des Blutes kommt es dann aufgrund der erhöhten Schwerwirkung zu einer direkten Schädigung von retinalen Gefäßendothelzellen. Dieser Mechanismus wird durch eine häufig bei Diabetikern gleichzeitig bestehende arterielle Hypertonie noch zusätzlich verstärkt.

Schließlich deuten verschiedene Untersuchungen auch auf einen Zusammenhang zwischen lmmunprozessen und der autonomen diabetischen Neuropathie hin. Es wird spekuliert, dass eine genetische Prädisposition immunologische Prozesse gegen Nervengewebe in Gang setzt, die durch Hyperglykämie oder Umweltfaktoren beschleunigt werden.

Aufgabe der vorliegenden Erfindung ist es deshalb, Mittel bereitzustellen, die zur Prophylaxe, Behandlung oder zur Unterstützung der Behandlung von Krankheiten geeignet sind, die mit einem erhöhten Gehalt an Glucose und/oder HbA_{1c} im Blut einhergehen, nämlich von Diabetes mellitus Typ 2, insbesondere diabetischer Retinopathie, Makulopathie und Neuropathie und des metabolischen Syndroms, und weitestgehend frei sind von unerwünschten Neben- und Wechselwirkungen.

Erfindungsgemäß wird diese Aufgabe gelöst durch die orale Verabreichung von α-linolensäurehaltigen Pflanzenölen.

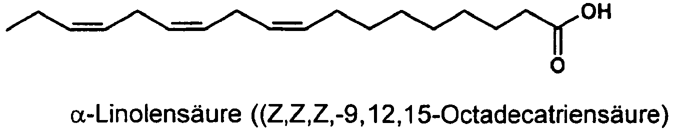

Die erfindungsgemäß verwendeten α-linolensäurehaltigen Pflanzenöle enthalten α-Linolensäure in gebundener Form in Form von Triglyceriden.

Das Öl aus den Samen von Perilla frutescens (Schwarznessel), nachfolgend als Perillaöl bezeichnet, enthält einen sehr hohen Gehalt an mehrfach ungesättigten essentiellen Fettsäuren in Form von Triglyceriden. Mit einem Anteil von etwa 50 - 60 % ist die zu den ω3-Fettsäuren zählende α-Linolensäure vorherrschend. Aus klinischen und experimentellen Untersuchungen ist bekannt, dass Fettsäuren dieses Typs über zahlreiche positive ernährungsphysiologische Eigenschaften verfügen. Durch Verfütterung von Perillaöl an Ratten wurde die Erkrankungshäufigkeit von Brust-, Dünn- und Dickdarmkrebs verringert. Bekannt sind außerdem entzündungs- und blutgerinnungshemmende Effekte. Eine bei humanen Probanden beobachtete Abnahme des Triglycerid- und Cholesterinspiegels im Blut sowie eine blutdrucksenkende Wirkung sind vermutlich wesentlich an der verringerten Rate von Herz-Kreislauferkrankungen beteiligt. Beschrieben wird außerdem, dass der Konsum von Perillaöl die Funktion des Immunsystems verbessert und die Lernfähigkeit erhöht. Essentielle Fettsäuren, zu denen auch α-Linolensäure gehört, sind Strukturbestandteile von zahlreichen Zellmembranen. Da diese Fettsäuren von Säugern nicht selbst synthetisiert werden können, müssen sie mit der Nahrung zugeführt werden.

Von mehrfach ungesättigten Fettsäuren, die zu der ω3- oder ω6-Familie gehören, ist bekannt, dass sie einen günstigen Einfluß auf den Energie-Stoffwechsel und die Glycogenogenese ausüben (S. D. Clarke, Br. J. Nutrition 83 (Suppl. 1), S59, 2000). Es gibt Daten, die darauf hindeuten, dass der Verzehr von ω3-ungesättigten Fettsäuren über eine Beeinflussung der Insulinresistenz den Fettstoffwechsel günstig beeinflussen kann. Gleichzeitig gibt es jedoch Versuchsergebnisse, die belegen, dass der Konsum von Fischöl, das reich an solchen Fettsäuren ist, die Insulinresistenz bei Patienten, die bereits an einem Diabetes Typ 2 leiden, noch zusätzlich verstärken kann. Aus der Literatur gibt es keinerlei Hinweise, dass die Einnahme von mehrfach ungesättigten Fettsäuren unmittelbar zu einer Senkung des erhöhten Nüchtern-Blutglucosespiegels und/oder der HbA_{1c}-Werte bei Patienten mit Diabetes mellitus Typ 2 führt. Insbesondere ist ein positiver Einfluß von Fischöl auf diese Laborparameter bisher nicht beschrieben worden (C. R. Sirtori, C. Galli, Biomed. Pharmacother. 56, 397, 2002; Y. Okuda et al., J. Diabetes Complications 10, 280, 1996). Die Empfehlung zum Verzehr von Fischöl für die diätetische Behandlung bei diesem Patientengut wird daher kontrovers diskutiert und in einigen Publikationen wird sogar vom Konsum von Fischöl bei Diabetikern abgeraten (U. N. Das, Prostaglandins Leukotriens Essent. Fatty Acids 63, 351, 2000; A. C. Rustan et al., Ann. New York Acad. Sci. 827, 310, 1997; P. N. Durrington et al., Heart 85, 544, 2001; C. R. Sirtori, C. Galli, Biomed. Pharmacother. 56, 397, 2002)..

Überraschenderweise wurde nun festgestellt, dass die orale Einnahme von α-linolensäurehaltigen Pflanzenölen, wobei die α-Linolensäure in gebundener Form in Form von Triglyceriden vorliegt, bei Patienten mit Diabetes mellitus Typ 2, also bei Menschen, deren Blut einen erhöhten Gehalt an Glucose und/oder HbA_{1c} aufweist, die genannten Gehalte zu senken vermag.

Beispiele für verwendbare α-linolensäurehaltige Pflanzenöle gemäß der vorliegenden Erfindung sind Perillaöl und Leinöl, bevorzugt ist Perillaöl. Die Erfindung ist jedoch nicht auf die genannten Pflanzenöle beschränkt.

In einer bevorzugten Ausführungsform der Erfindung werden α-linolensäurehaltige Pflanzenöle in gebundener Form in Form von Triglyceriden in Kombination mit Zimt oder einem Zimtextrakt verwendet.

Zimt ist eine der ältesten und begehrtesten Gewürzpflanzen der Welt. Zur Herstellung von Zimt wird die Rinde von Zweigen verschiedener Zimtbaumspezies, die zur Familie der Lorbeergewächse (lat. *Lauraceae*) gehören, verwendet. Die bekanntesten und am häufigsten verwendeten Arten sind der in China beheimatete Cinnamomum aromaticum Nees (Chinazimtbaum, Kassie) und der in Ceylon vorkommende Cinnamomum verum J. S. Presl (Ceylonesischer Zimtbaum, Kaneelbaum). Neben dem sogenannten Stangenzimt, bei dem es sich um die unverarbeitete, getrocknete und eingerollte Rinde handelt, wird Zimt im Handel auch im gemahlenen Zustand angeboten. Als Gewürz wird Zimt vor allem Süßspeisen, Gebäck und Glühwein zugesetzt. Aus den Abfällen wird das Zimtöl gewonnen, das zum Aromatisieren von Likören sowie als Duftstoff in der Parfümindustrie verwendet wird.

Zu den für Zimt beschriebenen biologischen Effekten zählen unter anderem vasodilatative, antithrombotische, spasmolytische, antiulzerative, antiallergische, antibakterielle, fungistatische und magensekretionsfördernde Eigenschaften (B. Qin et al. (2004) Horm. Metab. Res. 36, 119 - 125). In einer Reihe von Untersuchungen wird außerdem berichtet, daß wäßrige Extrakte aus Zimt die Glucose-Aufnahme und die Glykogensynthese in Adipozyten erhöhen (A. Khan et al. (1990) Biol. Trace Elem. Res. 29, 183; C. L. Broadhurst et al. (2000) J. Agric. Food Chem. 48, 849 - 852). Diese Effekte beruhen vermutlich auf einer verbesserten Insulin-Rezeptor-Signaltransduktion aufgrund einer Hemmung der Phosphotyrosin-Phophatase 1 B (J. Imparl-Radosevich et al. (1998) Horm. Res. 50, 177 - 182). Als wirksamkeitsrelevante Inhaltsstoffe wurden ein Hydroxychalcon, dessen Struktur nicht beschrieben wird, und andere Polyphenole postuliert (K. J. Jarvill-Taylor et al. (2001) J. Am. Coll. Nutr. 20, 327 - 336; R. A. Anderson et al. (2004) J. Agric. Food Chem. 52, 65 - 70).

Wie aus den vorausgehenden Ausführungen zu entnehmen ist, können die erfindungsgemäßen vorteilhaften Effekte von α-linolensäurehaltigen Pflanzenölen in gebundener Form in Form von Triglyceriden bei Patienten mit Diabetes mellitus Typ 2 und/oder metabolischem Syndrom deshalb optimal durch die gleichzeitige Verabreichung von Zimt oder Zimtextrakten ergänzt werden.

Bevorzugt verwendet werden auch Wirkstoffkombinationen aus α-linolensäurehaltigen Pflanzenölen, bevorzugt aus Perillaöl, und Zimt, wobei dieser bevorzugt in Form eines Extraktes und besonders bevorzugt in Form eines wässrigethanolischen Gesamtextraktes eingesetzt wird.

Extrakte aus Zimtrinde können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemp. bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck.

Zur Herstellung oral verabreichbarer Darreichungsformen wird ein α-linolensäurehaltiges Pflanzenöl ggf. unter Zusatz von Zimt oder einem Extrakt aus Zimtrinde und ggf. unter Zusatz von Hilfsstoffen wie z. B. Stabilisatoren, Füllmittel etc., in Kapseln abgefüllt. Insbesondere ist wegen der starken Oxidationsempfindlichkeit von ungesättigten Fettsäuren der Zusatz von Antioxidantien oder Radikalfängern, wie z. B. Vitamin C und/oder E zu den erfindungsgemäßen Pflanzenölen zu empfehlen. Die Dosierung erfolgt dabei so, dass pro Tag 1 bis 10 g, bevorzugt 2 bis 5 g α-linolensäurehaltiges Pflanzenöl zugeführt werden. Im Falle der Wirkstoffkombination mit Zimt oder Zimtextrakt sollen zusätzlich pro Tag 0.5 bis 20 g, bevorzugt 1 bis 10 g Zimt bzw. 0.1 bis 3 g, bevorzugt 0.3 bis 1.5 g Extrakt aus Zimtrinde zugeführt werden.

### Beispiele

### Beispiel 1

Das Pflanzenöl wird gemäß nachstehender Tabelle mit den Stabilisatoren und ggf. Hilfsstoffen gemischt und die erhaltene fließfähige Suspension wird mit einem geeigneten an sich bekannten Verfahren in Kapseln abgefüllt.

| Bestandteile | mg / Kapselfüllung (Durchschnittswerte) |
|---|---|
| Perillaöl (enthaltend ca. 58 Gew. % α-Linolensäure) | 445,0 |
| Vitamin C | 23,4 |
| Vitamin E | 5,5 |

### Beispiel 2

101 Patienten mit Diabetes mellitus Typ 2 erhielten 12 Wochen lang täglich 3 x 2 Kapseln gemäß Beispiel 1. Nach 4 Wochen, nach 8 Wochen und nach 12 Wochen wurden Laborwerte bestimmt (Tabelle).

| | Glucose (nüchtern) (mg / dl) | HbA_{1c} (mg / dl) |
|---|---|---|
| vor Behandlung | 142,63 ± 35,79 | 7,04 ± 1,02 |
| nach 4 Wochen | 115,58 ± 30,55* | nicht bestimmt |
| nach 8 Wochen | 118,38 ± 26,16* | 6,52 ± 0,89* |
| nach 12 Wochen | 117,91 ± 29,72* | 6,63 ± 0.90* |

| | | |
|---|---|---|
| *) p < 0.0001 (Irrtumswahrscheinlichkeit). | | |

Durch die Behandlung mit Kapseln gemäß Beispiel 1 trat bei den Patienten innerhalb von 4 Wochen (Glucose) bzw. 8 Wochen (HbA_{1c}) eine hochsignifikante Absenkung des Glucose- und HbA_{1c}-Gehalts im Blut ein.

### Beispiel 3: Trockenextrakt aus Zimtrinde (Gesamtextrakt)

150 g fein gemahlene Rinde vom Cinnamomum aromaticum werden dreimal mit je 1050 g Ethanol (60 Gew.-%) jeweils 1 h bei 50 °C gerührt. Nach dem Abkühlen auf RT wird filtriert (Seitz 1500). Die vereinigten Filtrate werden im Vakuum bei maximal 50 °C vom Ethanol befreit und gefriergetrocknet: 26,6 g (17,7 %) Trockenextrakt.

### Beispiel 4

Das Pflanzenöl wird gemäß nachstehender Tabelle mit dem Zimtextrakt, den Stabilisatoren und ggf. Hilfsstoffen gemischt und die erhaltene fließfähige Suspension wird mit einem geeigneten an sich bekannten Verfahren in Kapseln abgefüllt.

| Bestandteile | mg / Kapselfüllung (Durchschnittswerte) |
|---|---|
| Perillaöl (enthaltend ca. 58 Gew. % α-Linolensäure) | 400,0 |
| Zimtextrakt gemäß Beispiel 3 | 150,0 |
| Vitamin C | 23,4 |
| Vitamin E | 5,5 |

## Patentansprüche

1. Verwendung von α-linolensäurehaltigen Pflanzenölen zur Herstellung eines diätetischen Nahrungsmittels oder Arzneimittels zur Prophylaxe oder Behandlung von Krankheiten, die mit einem erhöhten Gehalt an Glucose und/oder HbA_{1c} im Blut einhergehen, nämlich Diabetes mellitus Typ 2, wobei die α-linolensäurehaltigen Pflanzenöle α-Linolensäure in gebundener Form in Form von Triglyceriden enthalten.

2. Verwendung nach Anspruch 1, wobei das Pflanzenöl Perillaöl ist.

3. Verwendung nach Anspruch 1, wobei das Pflanzenöl Leinöl ist.

4. Verwendung nach einem der Ansprüche 1 bis 3 zur Behandlung von diabetischer Retinopathie und Makulopathie.

5. Verwendung nach einem der Ansprüche 1 bis 3 zur Behandlung von diabetischer Neuropathie.

6. Verwendung von α-linolensäurehaltigen Pflanzenölen zur Herstellung eines diätetischen Nahrungsmittels oder Arzneimittels zur Prophylaxe oder Behandlung von Krankheiten, die mit einem erhöhten Gehalt an Glucose und/oder HbA_{1c} im Blut einhergehen, nämlich des metabolischen Syndroms, wobei die α-linolensäurehaltigen Pflanzenöle α-Linolensäure in gebundener Form in Form von Triglyceriden enthalten.

7. Verwendung nach Anspruch 6, wobei das Pflanzenöl Perillaöl ist.

8. Verwendung nach Anspruch 6, wobei das Pflanzenöl Leinöl ist.

9. Verwendung nach einem der Ansprüche 1 bis 8, in Kombination mit Zimt oder einem Extrakt aus Zimtrinde.

## Claims

1. Use of α-linolenic acid-containing vegetable oils for the preparation of a dietary food product or a medicament for the prophylaxis or treatment of diseases which involve an increased concentration of glucose and/or HbA_{1c} in the blood, namely diabetes mellitus of type 2, wherein the α-linolenic acid-containing vegetable oils contain α-linolenic acid in bound form in the form of triglycerides.

2. Use according to claim 1, wherein the vegetable oil is perilla oil.

3. Use according to claim 1, wherein the vegetable oil is linseed oil.

4. Use according to one of claims 1 to 3 for the treatment of diabetic retinopathy and maculopathy.

5. Use according to one of claims 1 to 3 for the treatment of diabetic neuropathy.

6. Use of α-linolenic acid-containing vegetable oils for the preparation of a dietary food product or a medicament for the prophylaxis or treatment of diseases which involve an increased concentration of glucose and/or HbA_{1c} in the blood, namely metabolic syndrome, wherein the α-linolenic acid-containing vegetable oils contain α-linolenic acid in bound form in the form of triglycerides.

7. Use according to claim 6, wherein the vegetable oil is perilla oil.

8. Use according to claim 6, wherein the vegetable oil is linseed oil.

9. Use according to one of claims 1 to 8, in combination with cinnamon or an extract from cinnamon bark.

## Revendications

1. Utilisation d'huiles végétales contenant de l'acide alpha-linolénique, servant à produire un produit alimentaire diététique ou un médicament pour prévenir ou traiter des maladies associées à un taux élevé en glucose et/ou en HbA_{1c} dans le sang, à savoir le diabète de type 2, sachant que les huiles végétales contenant de l'acide alpha-linolénique contiennent de l'acide alpha-linolénique présent sous la forme de triglycérides sous une forme liée.

2. Utilisation selon la revendication 1, sachant que l'huile végétale est de l'huile de périlla.

3. Utilisation selon la revendication 1, sachant que l'huile végétale est de l'huile de lin.

4. Utilisation selon l'une quelconque des revendications 1 à 3 servant à traiter la rétinopathie diabétique ou la maculopathie diabétique.

5. Utilisation selon l'une quelconque des revendications 1 à 3 servant à traiter la neuropathie diabétique.

6. Utilisation d'huiles végétales contenant de l'acide alpha-linolénique servant à produire un produit alimentaire diététique ou un médicament pour prévenir ou traiter des maladies associées à un taux élevé en glucose et/ou en HbA_{1c} dans le sang, à savoir le syndrome métabolique, sachant que les huiles végétales contenant de l'acide alpha-linolénique contiennent de l'acide alpha-linolénique sous la forme de triglycérides sous une forme liée.

7. Utilisation selon la revendication 6, sachant que l'huile végétale est de l'huile de périlla.

8. Utilisation selon la revendication 6, sachant que l'huile végétale est de l'huile de lin.

9. Utilisation selon l'une quelconque des revendications 1 à 8, en combinaison avec de la cannelle ou avec un extrait d'écorce de cannelle.
